# EUROPEAN PATENT SPECIFICATION

(11) **EP 1 593 340 B1**
(45) Date of publication and mention of the grant of the patent: **10.08.2011**
(21) Application number: 05009712.0
(22) Date of filing: 03.05.2005
(51) Int. Cl.: A61B 5/022

(54) **Cuff for blood pressure monitor**
Manschette für Blutdrucküberwachung
Brassard occlusif destiné à surveiller la pression sanguine

(30) Priority: 07.05.2004 JP 2004138923
(43) Date of publication of application: 09.11.2005
(73) Proprietor: Omron Healthcare Co., Ltd., Kyoto 615-0084 (JP)
(72) Inventor: Sano, Yoshihiko c/o Omron Healthcare Co.,Ltd., Kyoto-shi, Kyoto 615-0084 (JP); Karo, Hiromichi c/o Omron Healthcare Co.,Ltd., Kyoto-shi, Kyoto 615-0084 (JP); Kishimoto, Hiroshi c/o Omron Healthcare Co.,Ltd., Kyoto-shi, Kyoto 615-0084 (JP); Tanaka, Takahide c/o Omron Healthcare Co.,Ltd., Kyoto-shi, Kyoto 615-0084 (JP); Koike, Tadashi c/o Omron Healthcare Co.,Ltd., Kyoto-shi, Kyoto 615-0084 (JP); Eda, Kenji c/o Omron Healthcare Co.,Ltd., Kyoto-shi, Kyoto 615-0084 (JP); Miyata, Kiichiro c/o Omron Healthcare Co.,Ltd., Kyoto-shi, Kyoto 615-0084 (JP); Nakada, Tetsuya c/o Omron Healthcare Co.,Ltd., Kyoto-shi, Kyoto 615-0084 (JP)
(74) Representative: Kilian, Helmut

(56) References cited:
- EP-A- 1 224 907
- US-A- 3 517 661
- US-A- 3 906 937

## Description

### BACKGROUND OF THE INVENTION

### Field of the Invention

The present invention relates to a cuff for blood pressure monitor, in particular, a cuff for blood pressure monitor, which includes an elastic member for securing a fluid bag to a living body.

### Description of the Background Art

To measure a blood pressure value, generally, a cuff provided with a fluid bag for pressing an artery inside a living body is wound around a body surface, and arterial pressure pulse waves caused in the artery by inflation/deflation of the fluid bag are detected to measure the blood pressure value. Here, the cuff refers to a band-shaped structure having a bladder, which can be wound around a portion of a living body, for use in measurement of arterial pressure of an upper limb, a lower limb or the like by introducing fluid such as gas or liquid into the bladder. Thus, the cuff represents the concept including the fluid bag as well as means for winding the fluid bag around the living body. In particular, the cuff wound and fitted on an arm is called an arm band or a manchette.

The cuff includes a cover as a fastening band, which includes the fluid bag and serves to wind the fluid bag around the living body. In using the cuff for blood pressure monitor, it is necessary to unfailingly wind the cover around the living body so that the fluid bag is reliably secured to the living body. However, the infallible winding is not always guaranteed because a subject of the measurement is entrusted with the winding operation in the case of the conventional cuff for blood pressure monitor. Unless the infallible winding is assured, the measurement value is variable, which makes it difficult to accurately and stably measure the blood pressure value.

In order to realize the reliable winding of the cuff, a cuff for blood pressure monitor having a constitution in which the cover is provided with an elastic member called a curled elastic member in addition to the fluid bag is known (for example, see Japanese Laid-Open Patent Publication Nos. 2003-210423 and 61-238229). The curled elastic member formed from the elastic member serves to retain an annular state of the cuff and annularly wound to be thereby disposed on an outer side of the fluid bag so that the cuff is radially changeable in size. In using the cuff for blood pressure monitor having the curled elastic member, the fluid bag is pressed onto the living body and thereby secured thereto by the curled elastic member after the cuff is fitted on the arm, which enables the cuff to be repeatedly wound without fail.

However, the cuff for blood pressure monitor having the foregoing curled elastic member unfavorably entails a very complicated fitting operation. Therefore, in the case of a cuff for blood pressure monitor disclosed in EP 1224907 A forming the basis for the preamble of the independent claims, the winding operation is facilitated in such manner that one end of the annularly-wound curled elastic member is extended outward so that the other end of the curled elastic member is wound inward. Then, the extended end of the curled elastic member is hitched on the arm in the fitting operation and the curled elastic member is spread wide in the hitching state to be thereby wound around the arm.

However, when the cuff for blood pressure monitor disclosed in EP 1224907 A is used, the reliable winding state is not always guaranteed unless the user correctly understands the winding operation. Further, when the user carries out any erroneous fitting operation, there is a possibility that the fitting operation can be actually complicated rather than facilitated. When winding the cuff around an arm, it is necessary for the user to hold the cuff with the other hand. However, when the cuff is held in a wrong manner, the curled elastic member is spread with rather a difficulty, and the winding operation results in a remarkably complicated work particularly for the users who are elderly people, children and women who are weak in muscular strength and who have an upper arm of a large size which demands more strength for the curled elastic member to be spread unless the fitting operation is correctly carried out.

### SUMMARY OF THE INVENTION

An object of the present invention is to facilitate the fitting operation of the cuff to thereby allow the user to carry out a correct fitting operation and consequently achieve a correct and stable measurement of the blood pressure value.

A cuff for blood pressure monitor according to a first aspect of the present invention, as defined in claim 1, is wound around a living body to be fitted thereon and includes a fluid bag for pressing a living body, an elastic member wound annularly on the outside of the fluid bag and changeable in size in a radial direction, and a cover having a bag shape for including the fluid bag and the elastic member. The elastic member has an overlapping region in which one end and the other end thereof in a circumferential direction are overlapped with each other so that the one end is located on an inner side of the other end in a non-fitting state. The cuff has an indicator for showing a spot to be held by hand in the fitting operation at a position corresponding to a region opposing to the overlapping region of the elastic member and/or a region closer to the one end of the elastic member than the opposing region in the non-fitting state.

With this constitution, in the fitting operation in which the other end of the elastic member is hitched on the living body and the elastic member is spread wide in the hitching state to be thereby wound around the living body, the user is led to accurately hold by hand the position at which a large bending moment can be obtained. Accordingly, the elastic member can be spread with a relatively small force, which dramatically improves operability in the fitting operation. Therefore, the cuff can be reliably wound enabling the blood pressure value to be accurately and stably measured. Examples of the indicator include any object as far as it can merely indicate the spot to be held in the fitting operation.

A cuff for blood pressure monitor according to a second aspect of the present invention, as defined in claim 5, is wound around a living body to be fitted thereon and includes a fluid bag for pressing the living body, an elastic member wound annularly on the outside of the fluid bag and having one end and the other end in a circumferential direction, the elastic member being changeable in size in a radial direction, and a cover having a bag shape for including the fluid bag and the elastic member. The cuff has an indicator for showing a spot to be held by hand in the fitting operation at a position corresponding to a region closer to the one end than a central position of the elastic member in a circumferential direction extending from the one end to the other end of the elastic member.

With this constitution, in the fitting operation in which the other end of the elastic member is hitched on the living body and the elastic member is spread wide in the hitching state to be thereby wound around the living body, the user is led to accurately hold by hand the position at which the large bending moment can be obtained. Accordingly, the elastic member can be spread with a relatively small force, which dramatically improves the operability in the fitting operation. Therefore, the cuff can be reliably wound, and the blood pressure value can be accurately and stably measured. Examples of the indicator include any object as far as it can merely indicate the spot to be held in the fitting operation.

A cuff for blood pressure monitor according to a third aspect of the present invention, as defined in claim 6, is wound around a living body to be fitted thereon and includes a fluid bag for pressing the living body, an elastic member wound annularly on the outside of the fluid bag and having one end and the other end in a circumferential direction, the elastic member being changeable in size in a radial direction, and a cover having a bag shape for including the fluid bag and the elastic member. The elastic member has a bendable bending portion. The cuff has an indicator for showing a spot to be held by hand in the fitting operation at a position corresponding to a region closer to the one end of the elastic member than the bending portion of the elastic member.

With this constitution, in the fitting operation in which the other end of the elastic member is hitched on the living body and the elastic member is spread wide in the hitching state to be thereby wound around the living body, the user is led to accurately hold the correct position of the cuff by hand. Accordingly, the elastic member can be spread wide at the bending portion with a relatively small force, which dramatically improves the operability in the fitting operation. Therefore, the cuff can be reliably wound, and the blood pressure value can be accurately and stably measured. Examples of the indicator include any object as far as it can merely indicate the spot to be held in the fitting operation.

In the cuff for blood pressure monitor according to the first to third aspects of the present invention, a finger-hitching member attached to the surface of the cuff constitutes the indicator.

The cuff for blood pressure monitor in which the fitting operation is inexpensively and reliably improved can be provided when the finger-hitching member attached to the surface of the cuff is thus used as the indicator for showing the spot to be held by hand. The finger-hitching member represents an object on which a finger can be hitched unlike a sticker. When the finger-hitching member is used as the indicator, the cuff can be unfailingly held without the possibility of the slip of the finger in the fitting operation. The operability can be thereby further improved.

In the cuff for blood pressure monitor according to the first to third aspects of the present invention, preferably, the finger-hitching member has a recessed portion for receiving the finger in a surface thereof.

When the recessed portion is thus provided on the surface of the finger-hitching member, the finger can be surely fitted in the finger-hitching member, and the cuff can be thereby unfailingly held without the possibility of the slip of the finger in the fitting operation. The operability can be thereby further improved.

In the cuff for blood pressure monitor according to the first to third aspects of the present invention, preferably, the recessed portion is provided so as to extend in direction intersecting the circumferential direction of the elastic member extending from the one end to the other end thereof.

When the recessed portion is thus provided on the surface of the cuff at an angle with the longitudinal direction of the cuff, the user is led to hold the cuff in a correct manner. Thereby, the operability in the fitting operation is further improved.

In the cuff for blood pressure monitor according to the first to third aspects of the present invention, preferably, the finger-hitching member has a shape of a fingerstall.

When the finger-hitching member thus has the fingerstall shape, the finger can surely catch the finger-hitching member. Accordingly, the cuff can be reliably held without the possibility of the slip of the finger, and the operability in the fitting operation can be thereby further improved.

In the cuff for blood pressure monitor according to the first to third aspects of the present invention, preferably, the finger-hitching member is formed from elastomer.

When the finger-hitching member is thus formed from the elastomer, the finger can be surely fitted in the finger-hitching member. Thereby, the operability in the fitting operation can be further improved.

According to the present invention, the cuff can be far more easily wound leading the user to carry out the correct fitting operation. As a result, the blood pressure value can be more accurately and stably measured.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is an outline view of a cuff for blood pressure monitor according to a first embodiment, which is an embodiment not claimed, when the cuff is not fitted on an arm;
Fig. 2 is a sectional view of the cuff for blood pressure monitor according to the first embodiment when the cuff is not fitted on the arm;
Figs. 3 to 5 respectively show a method of fitting the cuff for blood pressure monitor according to the first embodiment;
Fig. 6 is a perspective view showing a state in which the cuff for blood pressure monitor according to the first embodiment is fitted on the arm;
Fig. 7 is a sectional view of a modification example of the cuff for blood pressure monitor according to the first embodiment when the cuff is not fitted on the arm;
Fig. 8 is an outline view of a cuff for blood pressure monitor according to a second embodiment, an embodiment of the present invention, when the cuff is not fitted on the arm;
Fig. 9 is a plan view of the cuff for blood pressure monitor according to the second embodiment when a cover is removed therefrom;
Fig. 10 is a sectional view of the cuff for blood pressure monitor according to the second embodiment when the cuff is not fitted on the arm;
Fig. 11 is an outline view of a cuff for blood pressure monitor according to a third embodiment, an embodiment not claimed, when the cuff is not fitted on the arm;
Fig. 12 is a sectional view of the cuff for blood pressure monitor according to the third embodiment when the cuff is not fitted on the arm;
Fig. 13 is an outline view of a cuff for blood pressure monitor according to a fourth embodiment, an embodiment not claimed, when the cuff is not fitted on the arm;
Fig. 14 is a sectional view of the cuff for blood pressure monitor according to the fourth embodiment when the cuff is not fitted on the arm;
Fig. 15 is a sectional view of the cuff for blood pressure monitor according to the fourth embodiment taken along line XV-XV in Fig. 14;
Figs. 16 to 18 respectively show a method of fitting the cuff for blood pressure monitor according to the fourth embodiment;
Fig. 19 is a perspective view showing a state in which the cuff for blood pressure monitor according to the fourth embodiment is fitted on the arm;
Figs. 20 to 22 respectively show another method of fitting the cuff for blood pressure monitor according to the fourth embodiment;
Fig. 23 is a sectional view of a cuff for blood pressure monitor according to a fifth embodiment, an embodiment not claimed, when the cuff is not fitted on the arm; and
Figs. 24 and 25 respectively show a method of fitting the cuff for blood pressure monitor according to the fifth embodiment.

### DESCRIPTION OF THE PREFERRED EMBODIMENTS

Hereinafter, preferred embodiments of the present invention are described in detail referring to the drawings.

### First Embodiment (not claimed)

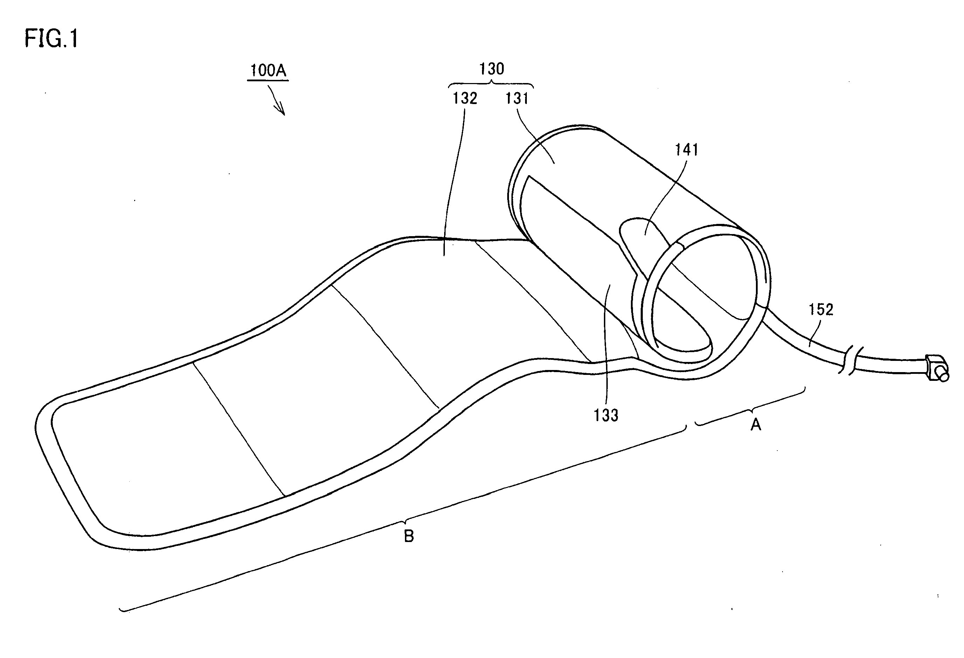

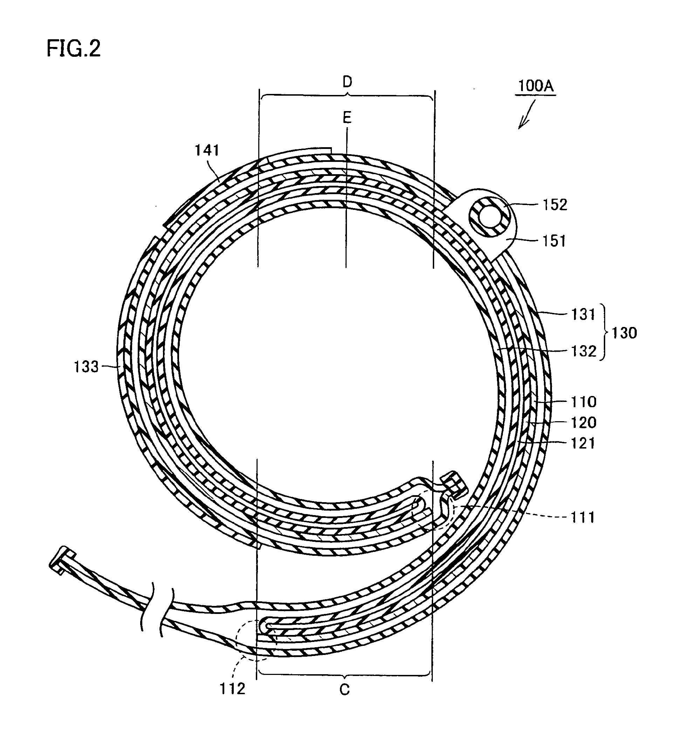

A cuff for blood pressure monitor 100A according to a first embodiment, an embodiment not claimed, is designed based on the intention that a subject him/herself fits the cuff on his/her upper left arm. As shown in Figs. 1 and 2, cuff 100A has an inserting portion A for inserting the upper left arm and a fastening portion B for fastening and securing the cuff with respect to the upper arm inserted into inserting portion A. A surface of cuff 100A is covered with a cover 130, which is a bag-shaped member made of a cloth. Provided within cover 130 are an air bag 120 serving as a fluid bag for pressing the upper arm, and a curled elastic member 110 disposed on an outer side of air bag 120 and serving as an elastic member. Curled elastic member 110 presses and secures air bag 120 with respect to a living body.

Air bag 120 has a bladder 121. Bladder 121 is connected to a rubber tube 152 via a nipple 151. Rubber tube 152 is connected to a blood pressure monitor main body (not shown). Bladder 121 of air bag 120 is pressurized or depressurized by means of a pressurizing pump, a negative pressure pump and the like incorporated in the blood pressure monitor main body when the blood pressure is measured, and air bag 120 is thereby inflated/deflated.

Curled elastic member 110 is annularly wound on the outside of air bag 120 so as to surround air bag 120. Curled elastic member 110 is formed from a resin member such as polypropylene and thereby adapted to retain its annular state and be changeable in size in a radial direction. Curled elastic member 110 has an overlapping region C in which one end 111 and the other end 112 thereof in a circumferential direction are overlapped with each other so that one end 111 is located on an inner side of the other end 112 in a non-fitting state.

Air bag 120 and curled elastic member 110 are housed inside of cover 130 formed in the bag shape from an outer cloth 131 and an inner cloth 132 which are stitched together. A velcro fastener 133 serving as a securing portion for fastening and securing cuff 100A with respect to the upper arm is bonded to or stitched on a predetermined position on an outer peripheral surface of cover 130.

When cuff 100A for blood pressure monitor according to this embodiment is in the non-fitting state, a sticker 141 as an indicator for showing a spot to be held by hand in the fitting operation is bonded to a surface of outer cloth 131 of cover 130 at a position corresponding to a region D opposing to overlapping region C of curled elastic member 110 and a region closer to one end 111 of curled elastic member 110 than opposing region D. In cuff 100A for blood pressure monitor according to this embodiment shown in the drawing, sticker 141 is bonded to the surface of cuff 100A such that it is arranged across a position corresponding to one of the circumferential ends of opposing region D that is closer to one end 111 of curled elastic member 110. As shown in Fig. 1, sticker 141 extends on the surface of cuff 100A in a direction intersecting a longitudinal direction of cuff 100A.

With this constitution, the spot to be held by hand in the fitting operation can be explicitly indicated by sticker 141. Therefore, a user can proceed with a correct fitting operation using sticker 141, which is the indicator, as a mark. As a result, the winding operation of the cuff can be largely facilitated, which enables a correct fitting state to be repeatedly achieved in each measurement. Thus, the blood pressure value can be correctly and stably measured.

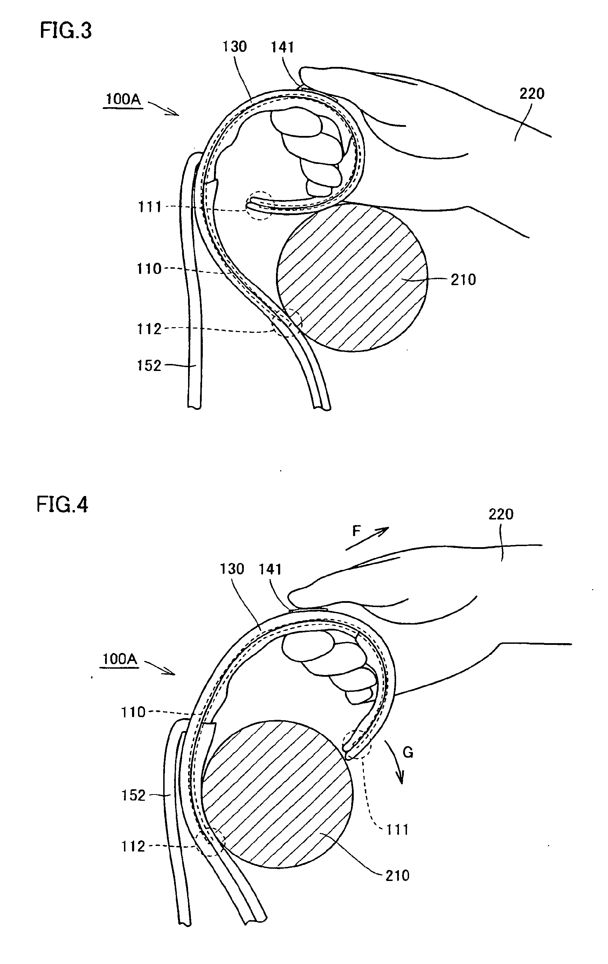

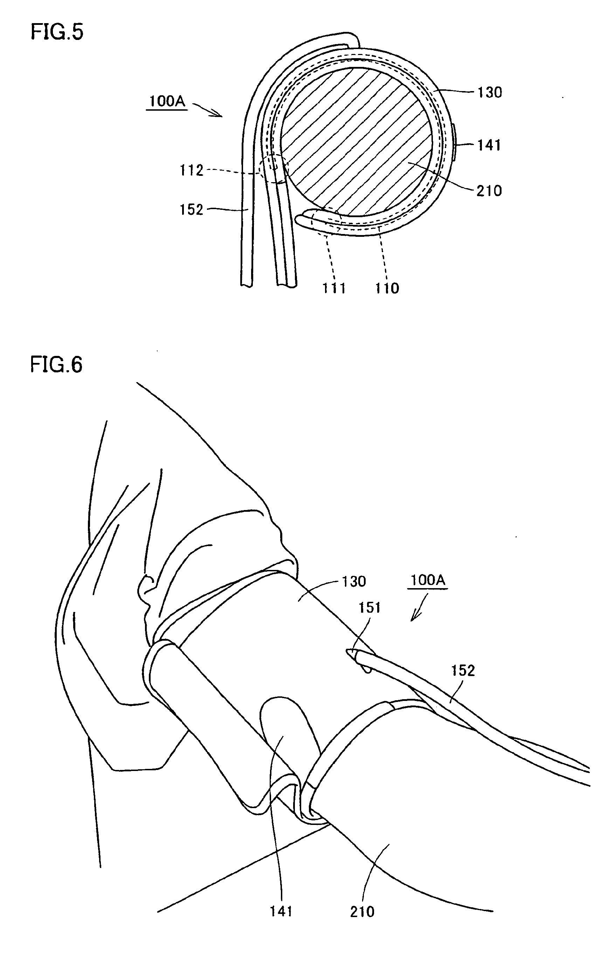

Referring to Figs. 3 to 5, details of the fitting operation in the case of using cuff 100A for blood pressure monitor according to this embodiment are described below following each step. Further, the correct fitting state in the case of using cuff 100A for blood pressure monitor according to this embodiment is described referring to Fig. 6.

As shown in Fig. 3, the user retains cuff 100A for blood pressure monitor so as to sandwich a side part thereof with his/her right hand 220. At that time, the user places a thumb of right hand 220 on an outer peripheral surface of cuff 100A to thereby cover and hide sticker 141 with the thumb using sticker 141 bonded to the outer peripheral surface of cuff 100A for blood pressure monitor as the mark, and further places the rest of his/her fingers other than the thumb on an inner peripheral surface of cuff 100A. The user, then, hitches the other end 112 of curled elastic member 110 on an outer side of an upper arm of his/her left hand 210.

Next, as shown in Fig. 4, the user lifts right hand 220 in a direction of an arrow F in the drawing to thereby spread curled elastic member 110 wide and open cuff 100A maintaining the state in which the other end 112 of curled elastic member 110 is hitched on the outer side of the upper arm of left hand 210, and also shifts right hand 220 in a direction of an arrow G in the drawing to thereby fit an end portion of cuff 100A corresponding to one end 111 side of curled elastic member 110 in an inner side of the upper arm of left hand 210, as a result of which a state shown in Fig. 5 arrives.

Through the foregoing steps, the upper arm of left hand 210 can be inserted into inserting portion A of cuff 100A, and further, cuff 100A can be secured to the upper arm of left hand 210 with an appropriate fastening force in response to a radial elasticity retaining force of curled elastic member 110. Then, fastening portion B of cuff 100A is wound around the upper arm of left hand 210 over inserting portion A and fastened and secured with respect to the upper arm by means of velcro fastener 133. In the foregoing manner, a fitting state shown in Fig. 6 is realized.

When the fitting operation in which cuff 100A is fitted by hitching the other end 112 of curled elastic member 110 on the outer side of the upper arm is carried out, a central position of air bag 120 in circumferential and width directions thereof is disposed at a position corresponding to an arterial position on the upper arm of left hand 210 in the fitting state shown in Fig. 6. As a result, air bag 120 can be inartificially positioned at a suitable position, and the blood pressure value can be thereby accurately and stably measured.

As described so far, in cuff 100A for blood pressure monitor according to this embodiment, sticker 141 as the indicator for showing the spot to be held by hand in the fitting operation is bonded to the surface of outer cloth 131 of cover 130 at the position corresponding to region D opposing to overlapping region C of curled elastic member 110 and the region closer to one end 111 of curled elastic member 110 than opposing region D. Therefore, the user can be led to hold cuff 100A using sticker 141 as the mark in the fitting operation, and further, guided to accurately hold a position at which a large bending moment can be obtained. Curled elastic member 110 can be thereby greatly changed in size with a small strength in the fitting operation, which enables curled elastic member 110 to be spread with a relatively small strength. The fitting operation can be thus more efficiently operable, wherein cuff 100A can be reliably wound and the blood pressure value can be consequently measured in an accurate and stable manner.

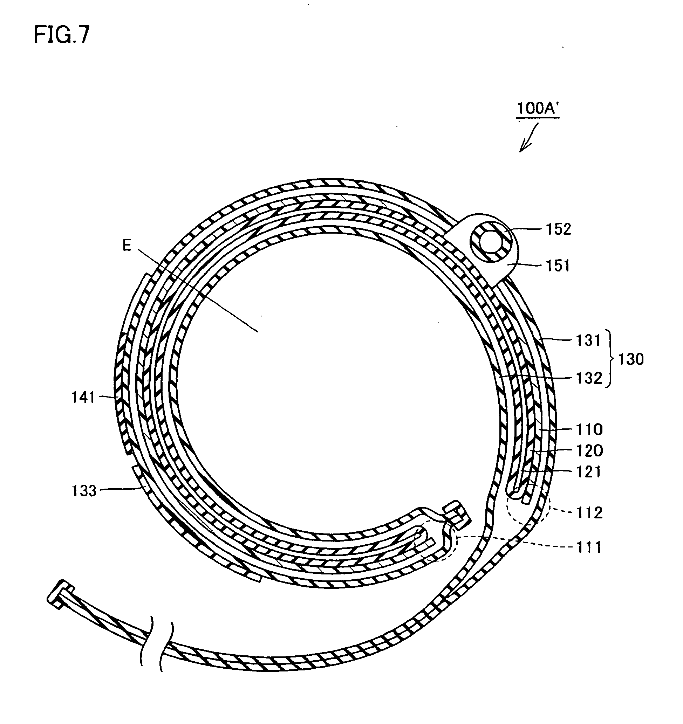

In a cuff 100A' for blood pressure monitor shown in Fig. 7, which is different from cuff 100A recited earlier, curled elastic member 110 does not have the overlapping region and is provided with a clearance between one end 111 and the other end 112 in the circumferential direction of curled elastic member 110 in the non-fitting state. When cuff 100A' for blood pressure monitor constituted as described is to be fitted, the fitting operation in which the other end 112 of curled elastic member 110 is hitched on the arm and the cuff is wound around the arm while the curled elastic member is spread wide in the hitching state can be carried out in the same manner as in cuff 100A.

In the case of using cuff 100A' for blood pressure monitor shown in Fig. 7, sticker 141 serving as the indicator for showing the spot to be held by hand in the fitting operation is bonded to the surface of outer cloth 131 of cover 130 at a position corresponding to a region closer to one end 111 than a central position E of curled elastic member 110 in the circumferential direction extending from one end 111 to the other end 112 thereof. When sticker 141 is bonded to the position, the user can be guided to accurately hold the spot at which the large bending moment can be obtained in the fitting operation, and curled elastic member 110 can be thereby spread with a relatively small force. As a result, cuff 100A' can be reliably wound, which successfully achieves an accurate and stable measurement of the blood pressure value.

### Second Embodiment

A cuff 100B for blood pressure monitor according to a second embodiment, an embodiment of the present invention, is designed so that the subject can fit the cuff on his/her upper left arm him/herself in the same manner as in cuff 100A according to the first embodiment. Therefore, any component in this embodiment, which is the same as in cuff 100A for blood pressure monitor according to the first embodiment, is simply provided with the same reference numeral and is not described here again.

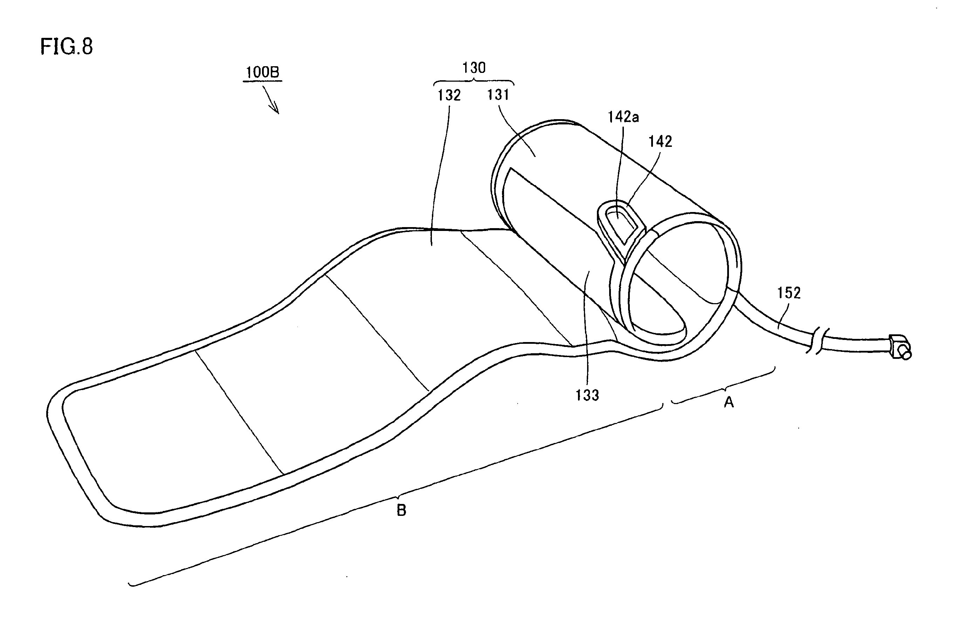

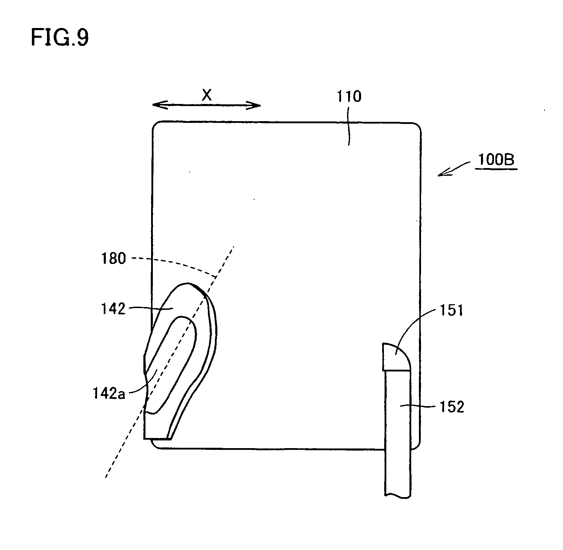

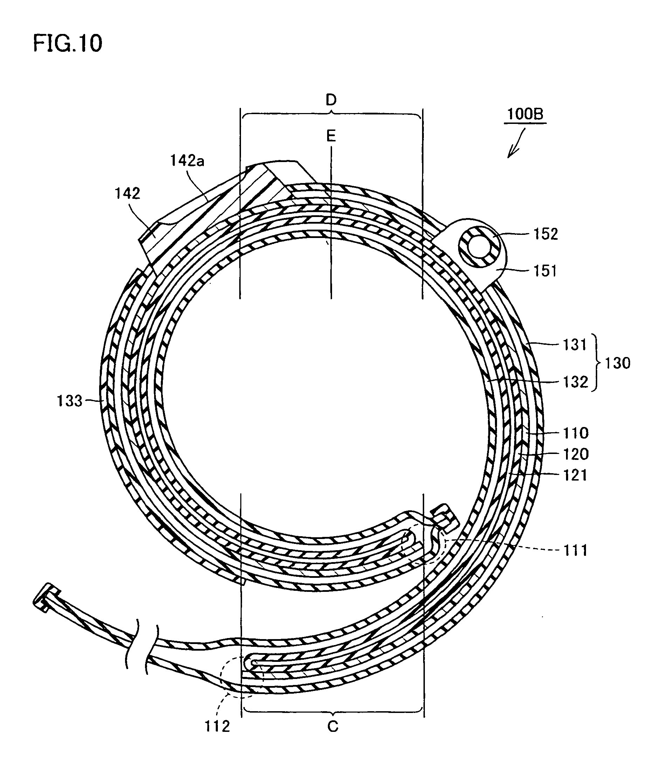

As shown in Figs. 8 to 10, cuff 100B for blood pressure monitor according to this embodiment, which is different to cuff 100A for blood pressure monitor according to the first embodiment, employs a finger-hitching member 142 formed from elastomer serving as the indicator for showing the spot to be held by hand in the fitting operation. As shown in Fig. 9, finger-hitching member 142 is secured to an outer peripheral surface of curled elastic member 110 by means of an adhesive or the like. Outer cloth 131 of cover 130 at a position corresponding to finger-hitching member 142 is slashed so that a surface of finger-hitching member 142 can be exposed.

In the case of cuff 100B for blood pressure monitor according to this embodiment, as in cuff 100A for blood pressure monitor according to the first embodiment, finger-hitching member 142, which serves as the indicator for showing the spot to be held by hand in the fitting operation, is attached to a surface of cuff 100B at the position corresponding to region D opposing to overlapping region C of curled elastic member 110 and the region closer to one end 111 of curled elastic member 110 than opposing region D in the non-fitting state.

Finger-hitching member 142 has a recessed portion 142a so as to increase a fitting property with respect to the thumb of the right hand. Cuff 100B can be surely held by hand without causing the slip of the finger in the fitting operation by providing recessed portion 142a on the surface of finger-hitching member 142 as described. Further, recessed portion 142a extends in a direction (direction shown in a broken line 180 in Fig. 9) intersecting the circumferential direction of curled elastic member 110 extending from one end 111 to the other end 112 thereof (direction shown with an arrow X in Fig. 9). When recessed portion 142a is thus provided on the surface at an angle with the longitudinal direction of cuff 100B, cuff 100B can be held in an accurate manner, which further improves the operability in the fitting operation.

According to the constitution described so far, the user can be guided to hold cuff 100B using finger-hitching member 142 as the mark in the fitting operation, which further leads the user to accurately hold the position at which the large bending moment can be obtained in the same manner as in cuff 100A for blood pressure monitor according to the first embodiment. Accordingly, curled elastic member 110 can be greatly changed in size with a small force in the fitting operation and thereby spread with a relatively small force. The operability in the fitting operation is significantly improved, and the cuff can be correspondingly reliably wound. As a result, the blood pressure value can be accurately and stably measured. The use of the finger-hitching member as the indicator as recited in this embodiment prevents the slip of the finger in contrast to the case of using the sticker, wherein the fitting operation can be further facilitated.

### Third Embodiment (not claimed)

A cuff 100C for blood pressure monitor according to a third embodiment is designed so that the subject can fit the cuff on his/her left upper arm him/herself in the same manner as in cuff 100A according to the first embodiment. Therefore, any component in this embodiment, which is the same as in cuff 100A for blood pressure monitor according to the first embodiment, is simply provided with the same reference numeral and is not described here again.

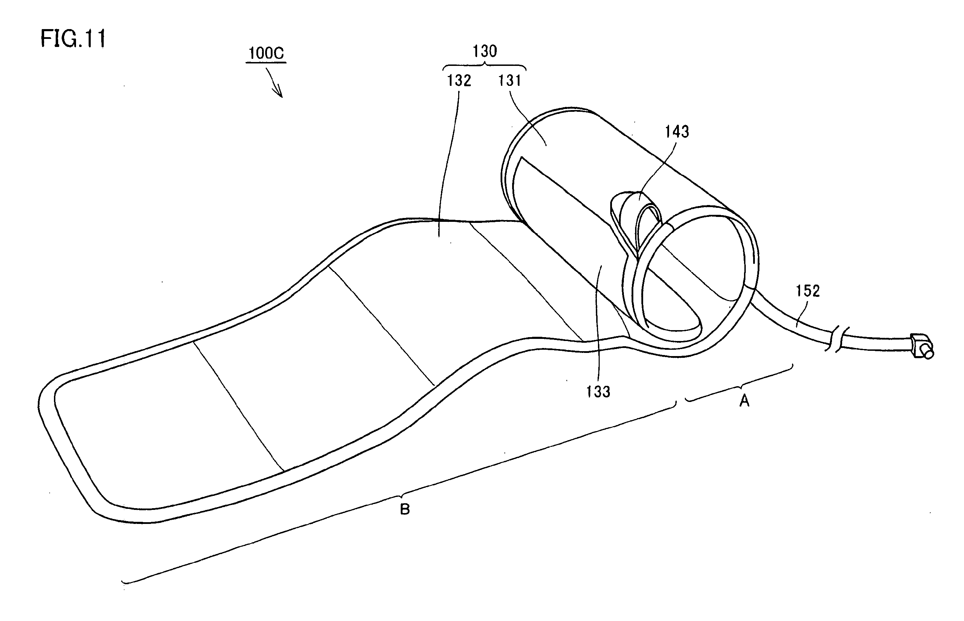

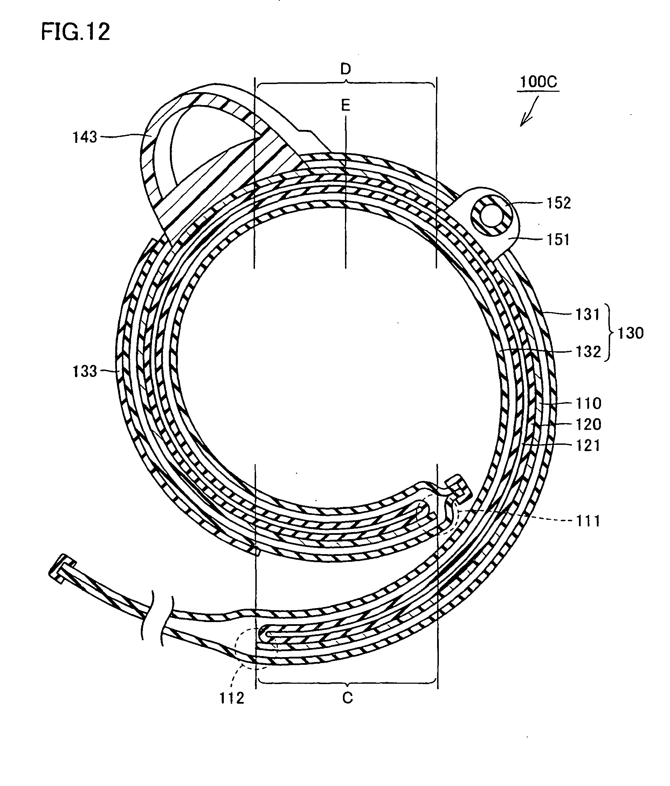

As shown in Figs. 11 and 12, cuff 100C for blood pressure monitor according to this embodiment, which is different to cuff 100A for blood pressure monitor according to the first embodiment, employs a finger-hitching member 143 having a fingerstall shape and serving as the indicator for showing the spot to be held by hand in the fitting operation. Finger-hitching member 143 is attached to, for example, the surface of outer cloth 131 of cover 130 by means of stitching, bonding or the like.

In the case of cuff 100C for blood pressure monitor according to this embodiment, in the same manner as in cuff 100A for blood pressure monitor according to the first embodiment, finger-hitching member 143 having the fingerstall shape, which serves as the indicator for showing the spot to be held by hand in the fitting operation, is attached to a surface of cuff 100C at the position corresponding to region D opposing to overlapping region C of curled elastic member 110 and the region closer to one end 111 of curled elastic member 110 than opposing region D in the non-fitting state. Further, as shown in Fig. 11, finger-hitching member 143 having the fingerstall shape extends in a direction intersecting a longitudinal direction of cuff 100C on the surface of cuff 100C.

When finger-hitching member 143 has the fingerstall shape as in the case of cuff 100C for blood pressure monitor according to this embodiment, the finger can be reliably hitched on finger-hitching member 143, and cuff 100C can be unfailingly held by hand without the slip of the finger in the fitting operation. With this constitution described so far, in the same manner as in cuff 100A for blood pressure monitor according to the first embodiment, the user can be guided to hold cuff 100C using finger-hitching member 143 as the mark in the fitting operation, which further leads the user to accurately hold the position at which the large bending moment can be obtained. Accordingly, curled elastic member 110 can be greatly changed in size with a small force and thereby spread with a relatively small force in the fitting operation. The operability in the fitting operation is significantly improved, and the cuff can be reliably wound. As a result, the blood pressure value can be accurately and stably measured. The use of the finger-hitching member as the indicator as recited in this embodiment prevents the slip of the finger in contrast to the case of using the sticker, wherein the fitting operation can be further facilitated.

### Fourth Embodiment (not claimed)

A cuff 100D for blood pressure monitor according to a fourth embodiment is designed so that the subject can fit the cuff on his/her left upper arm him/herself in the same manner as in cuff 100A according to the first embodiment. Therefore, any component in this embodiment, which is the same as in cuff 100A for blood pressure monitor according to the first embodiment, is simply provided with the same reference numeral and is not described here again.

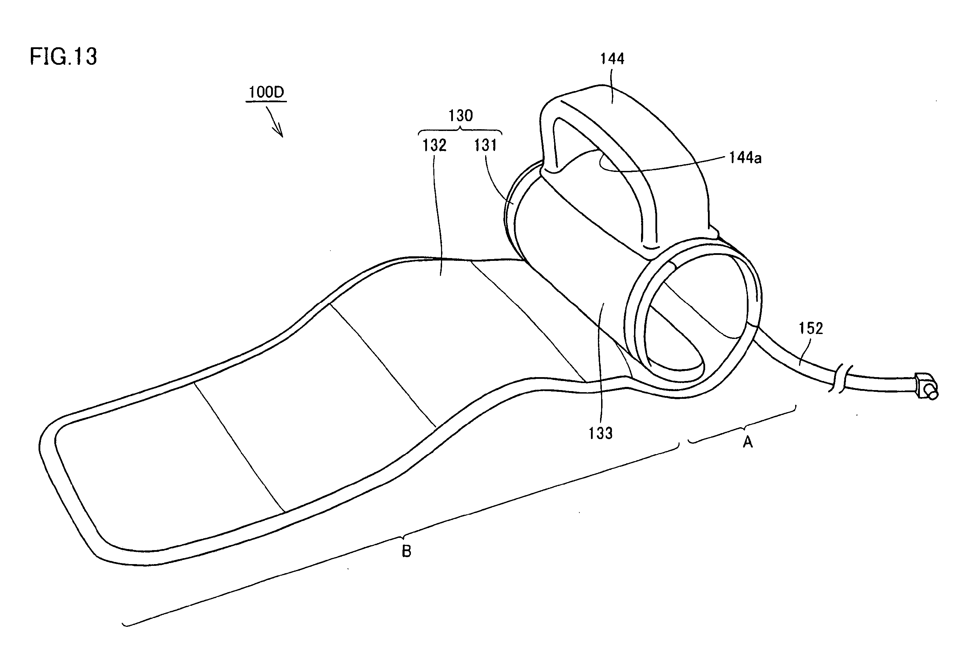

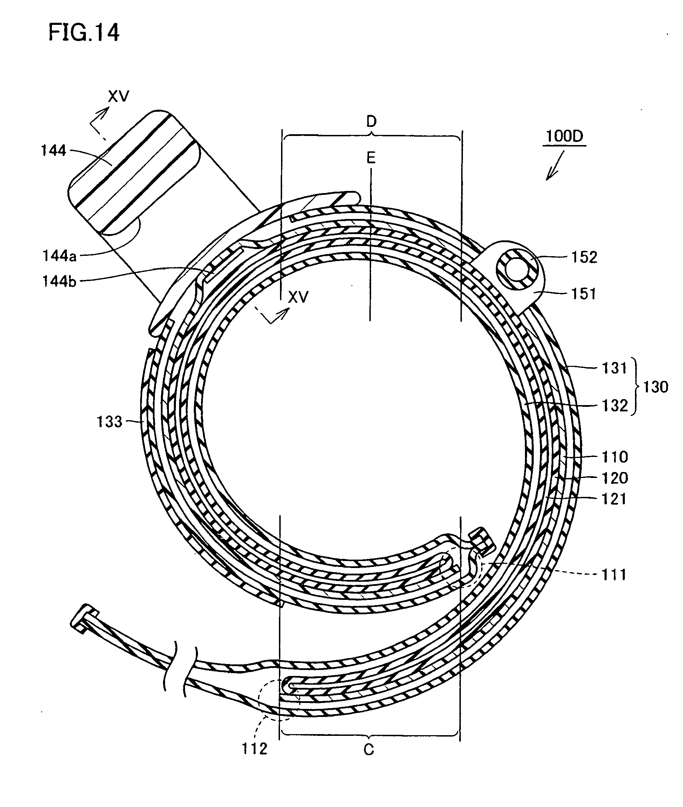

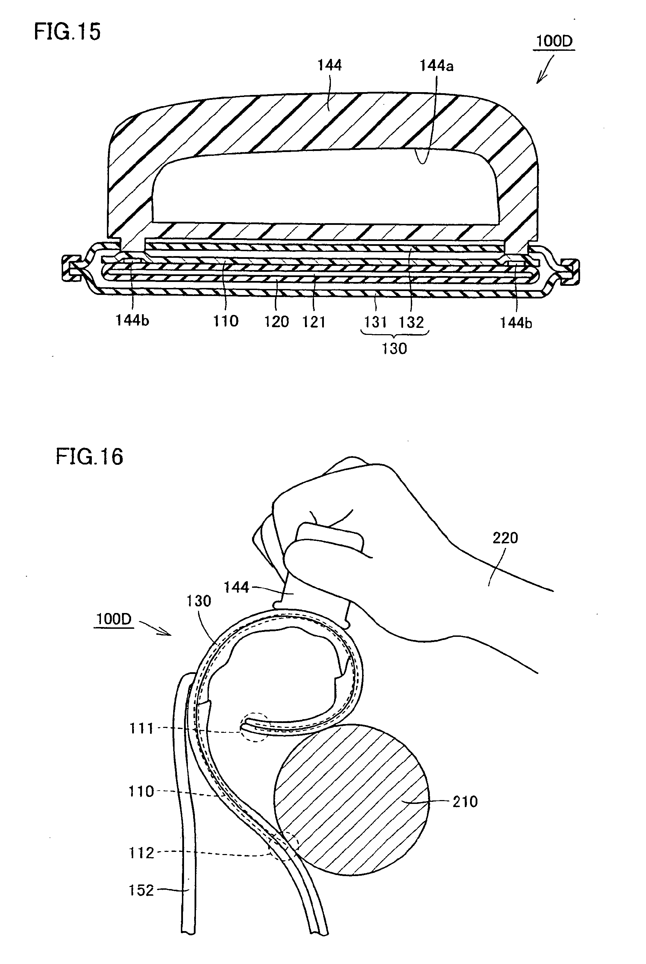

As shown in Figs. 13 to 15, cuff 100D for blood pressure monitor according to the fourth embodiment, which is different from cuff 100A for blood pressure monitor according to the first embodiment, employs a grip portion 144 as the indicator for showing the spot to be held in the fitting operation. As shown in Figs. 14 and 15, grip portion 144 is secured to the outer peripheral surface of curled elastic member 110 by means of a flat machine screw 144b serving as a securing portion. Grip portion 144 has an opening 144a into which the four fingers other than the thumb of the right hand can be inserted.

In the case of cuff 100D for blood pressure monitor according to this embodiment, in the same manner as in cuff 100A for blood pressure monitor according to the first embodiment, grip portion 144, which serves as the indicator for showing the spot to be held by hand in the fitting operation, is attached to a surface of cuff 100D at the position corresponding to region D opposing to overlapping region C of curled elastic member 110 and the region closer to one end 111 of curled elastic member 110 than opposing region D in the non-fitting state.

According to the foregoing constitution, the spot to be held by hand in the fitting operation can be explicitly shown by grip portion 144, and the user can thereby follow the accurate fitting operation by holding grip portion 144 as the indicator. As a result, the winding operation of the cuff can be significantly facilitated, which enables the correct fitting state to be continuously repeated in each measurement. The blood pressure value can be thus measured with an accuracy and stability.

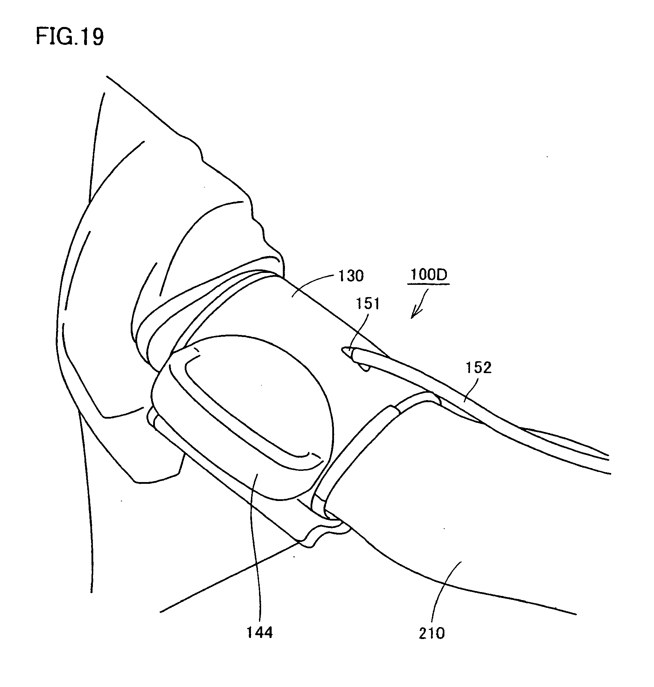

Below are described specifics of the fitting operation in the case of using cuff 100D for blood pressure monitor according to this embodiment following each step with reference to Figs. 16 to 18, and the accurate fitting state in the case of using cuff 100D for blood pressure monitor according to this embodiment with reference to Fig. 19.

First, as shown in Fig. 16, the user inserts the four fingers other than the thumb of right hand 220 into opening 144a of grip portion 144 attached to the surface of cuff 100D for blood pressure monitor to thereby hold grip portion 144, and hitches the other end 112 of curled elastic member 110 on the outer side of the upper arm of left hand 210.

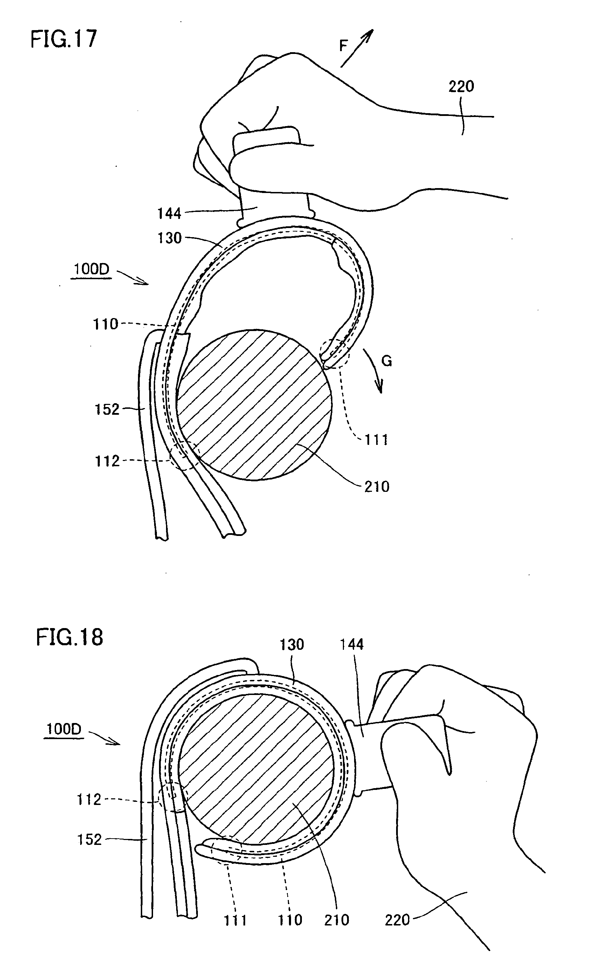

Next, as shown in Fig. 17, the user maintains the state in which the other end 112 of curled elastic member 110 is hitched on the outer side of the upper arm of left hand 210, while lifting right hand 220 in a direction of an arrow F to thereby spread curled elastic member 110 wide and open cuff 100D. The user, then, shifts right hand 220 in a direction of an arrow G and engages an end portion of cuff 100D corresponding to one end 111 side of curled elastic member 110 with the inner side of the upper arm of left hand 210, as a result of which a state shown in Fig. 18 is generated.

Through the foregoing steps, the upper arm of left hand 210 can be inserted into inserting portion A of cuff 100D, and cuff 100D can be secured to the upper arm of left hand 210 with an appropriate fastening force in response to the radial elasticity retaining force of curled elastic member 110. Then, fastening portion B of cuff 100D is wound around the upper arm of left hand 210 over inserting portion A and fastened and secured with respect to the upper arm by means of velcro fastener 133 mentioned earlier. In the foregoing manner, a fitting state shown in Fig. 19 can be realized.

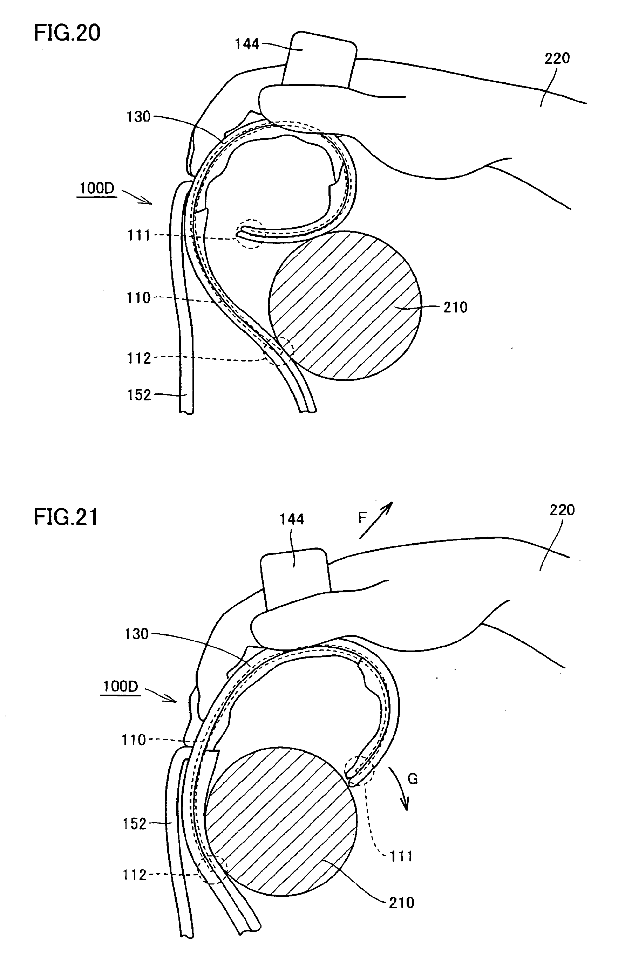

In the case of cuff for blood pressure monitor 100D as recited in this embodiment, the fitting operation according to another fitting method as follows, apart from the foregoing fitting operation, can be performed. Below are described, referring to Figs. 20 to 22, specifics of the fitting operation in which the another method of fitting cuff 100D for blood pressure monitor according to this embodiment is adopted following each step.

First, as shown in Fig. 20, the user inserts the four fingers other than the thumb of right hand 220 into opening 144a of grip portion 144 attached to the surface of cuff 100D for blood pressure monitor to thereby hold grip portion 144 and places right hand 220 on cuff 100D so that a palm of right hand 220 faces the outer peripheral surface of cuff 100D. The user, then, hitches the other end 112 of curled elastic member 110 on the outer side of the upper arm of left hand 210.

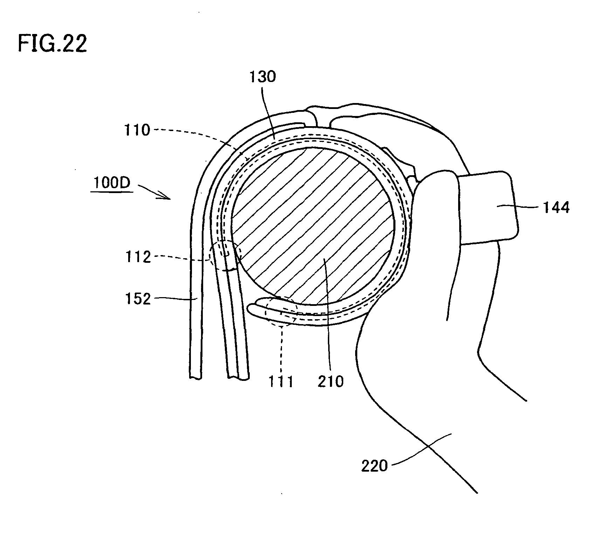

Next, as shown in Fig. 21, the user maintains the state in which the other end 112 of curled elastic member 110 is hitched on the outer side of the upper arm of left hand 210, while lifting right hand 220 in a direction of an arrow F to thereby spread curled elastic member 110 wide and open cuff 100D. The user, then, shifts right hand 220 in a direction of an arrow G and engages the end portion of cuff 100D corresponding to one end 111 side of curled elastic member 110 with the inner side of the upper arm of left hand 210, as a result of which the state shown in Fig. 22 is generated.

Through the foregoing steps, the upper arm of left hand 210 can be inserted into inserting portion A of cuff 100D, and cuff 100D can be secured to the upper arm of left hand 210 with an appropriate fastening force in response to the radial elasticity retaining force of curled elastic member 110. Then, fastening portion B of cuff 100D is wound around the upper arm of left hand 210 over inserting portion A and fastened and secured with respect to the upper arm by means of velcro fastener 133 mentioned earlier. In the foregoing manner, the fitting state shown in Fig. 19 can be realized.

In performing one of the fitting operations in which the cuff is fitted by hitching the other end 112 of curled elastic member 110 on the upper arm, the central position in the circumferential and width directions of air bag 120 is disposed at the position corresponding to the arterial position on the upper arm of left hand 210 in the fitting state shown in Fig. 19. As a result, air bag 120 can be inartificially positioned at an appropriate position enabling the blood pressure value to be accurately and stably measured.

As so far described, in the case of cuff 100D for blood pressure monitor according to this embodiment, grip portion 144, which serves as the indicator for showing the spot to be held by hand in the fitting operation, is provided on the surface of outer cloth 131 of cover 130 at the position corresponding to region D opposing to overlapping region C of curled elastic member 110 and the region closer to one end 111 of curled elastic member 110 than opposing region D in the non-fitting state. Accordingly, the user can be led to hold grip portion 144 in the fitting operation and further led to accurately hold the position at which the large bending moment can be obtained. Therefore, curled elastic member 110 can be greatly changed in size with a small force in the fitting operation and can be consequently spread with a relatively small force. The cuff can be then reliably wound along with the dramatically improved operability in the fitting operation, as a result of which the blood pressure value can be accurately and stably measured.

### Fifth Embodiment (not claimed)

A cuff 100E for blood pressure monitor according to a fifth embodiment, is designed in such manner that the subject can fit the cuff on his/her left upper arm him/herself in the same manner as in cuff 100D according to the fourth embodiment. Therefore, any component in this embodiment, which is the same as in cuff 100D for blood pressure monitor according to the fourth embodiment, is simply provided with the same reference numeral and is not described here again.

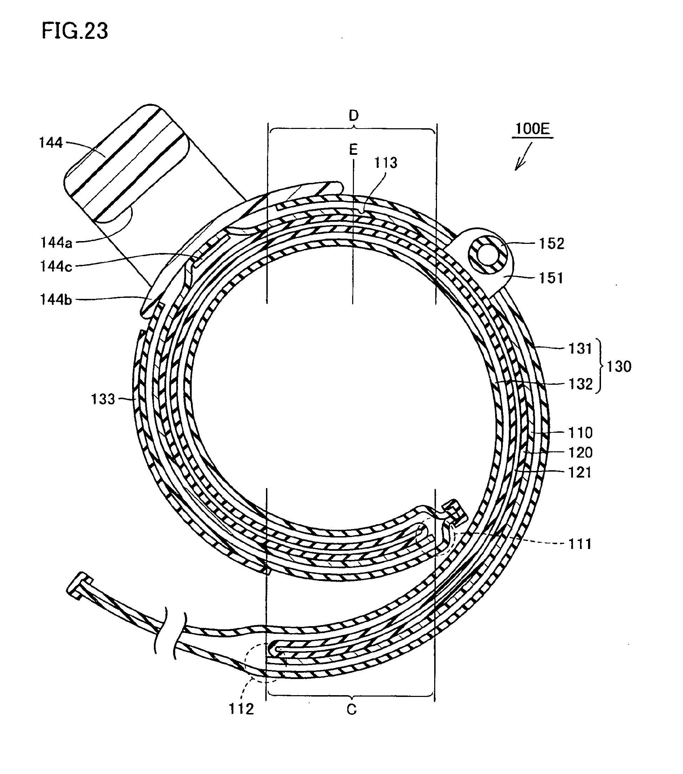

As shown in Fig. 23, cuff for blood pressure monitor 100E according to this embodiment includes grip portion 144, which serves as the indicator for showing the spot to be held by hand in the fitting operation, on a surface thereof in the same manner as in cuff for blood pressure monitor 100D according to the fourth embodiment. Cuff 100E for blood pressure monitor according to this embodiment further includes a bending portion 113 at a predetermined position on the other end 112 side relative to a position where grip portion 144 is provided in curled elastic member 110, which does not conform to the constitution of cuff 100D for blood pressure monitor according to the fourth embodiment. More specifically, grip portion 144 is disposed on one end 111 side relative to bending portion 113 in the circumferential direction of curled elastic member 110. Bending portion 113 is a portion arranged to be more easily bendable than any other part of curled elastic member 110. In cuff 100E for blood pressure monitor according to this embodiment, bending portion 113 is formed from a notch provided in the outer peripheral surface of curled elastic member 110.

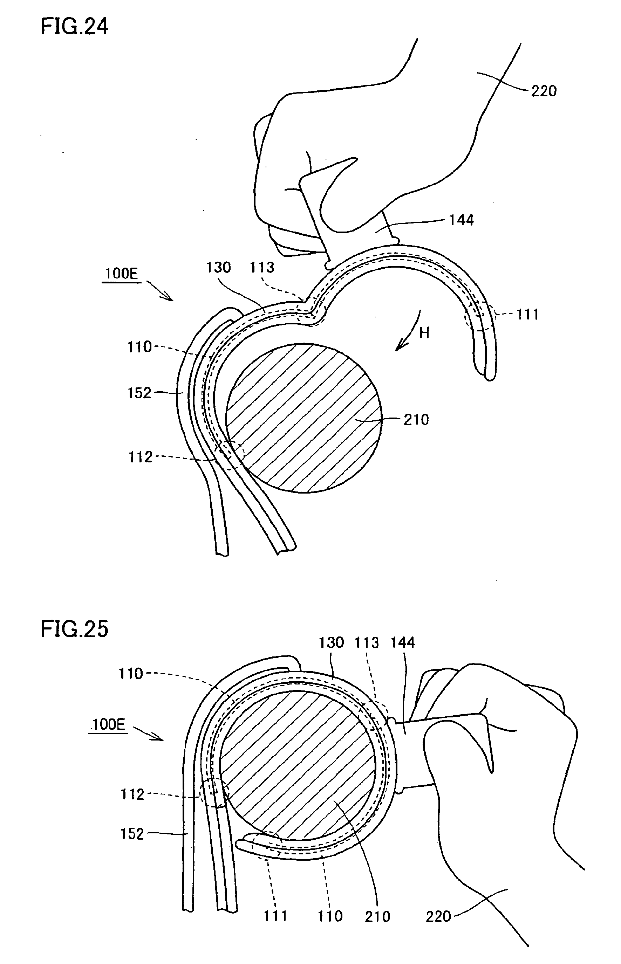

Below are described specifics of the fitting operation in the case of using cuff 100E for blood pressure monitor following each step with reference to Figs. 24 and 25.

First, as shown in Fig. 24, the user inserts the four fingers other than the thumb of right hand 220 into opening 144a of grip portion 144 attached to the surface of cuff for blood pressure monitor 100E to thereby hold grip portion 144, and hitches the other end 112 of curled elastic member 110 on the outer side of the upper arm of left hand 210. At that time, curled elastic member 110 is largely bent at bending portion 113 by a small force applied to grip portion 144, and cuff 100E is consequently opened wide as shown in the drawing.

Next, as shown in Fig. 25, the user maintains the state in which the other end 112 of curled elastic member 110 is hitched on the outer side of the upper arm of left hand 210, while shifting right hand 220 downward in a direction of an arrow H to thereby engages an end portion of cuff 100E with the inner side of the upper arm of left hand 210.

Through the foregoing steps, the upper arm of left hand 210 can be inserted into inserting portion A of cuff 100E, and cuff 100E can be secured to the upper arm of left hand 210 with an appropriate fastening force in response to the radial elasticity retaining force of curled elastic member 110. Then, fastening portion B of cuff 100E is wound around the upper arm of left hand 210 over inserting portion A and fastened and secured with respect to the upper arm by means of velcro fastener 133 mentioned earlier. In the foregoing manner, a fitting state, which conforms to the fitting state according to the fourth embodiment shown in Fig. 19, can be realized.

With this constitution, the user can hold grip portion 144 as the indicator to thereby follow the accurate fitting operation because the spot to be held by hand in the fitting operation can be explicitly shown by grip portion 144. In addition to the foregoing advantage, curled elastic member 110 can be spread wide with a small force when the other end 112 of curled elastic member 110 is hitched on the outer side part of the left arm because bending portion 113 is more easily bendable than any other part of curled elastic member 110 as described earlier. As a result, the winding operation of the cuff is largely facilitated, and the correct fitting state can be repeatedly achieved in each measurement. The blood pressure value can be thereby accurately and stably measured.

A constitution of bending portion 113 is subject to various modifications. For example, bending portion 113 may be formed from the thinned portion resulting from the notch made into curled elastic member 110 as described or formed from a slit provided in the width direction of curled elastic member 110. As more alternatives, bending portion 113 may be constituted in such manner that curled elastic member 110 is divided into two parts which are connected by an elastic member such as a spring, or may be designed in such manner that curled elastic member 110 is left unprocessed and grip portion 144 is formed from a member having a larger rigidity than that of curled elastic member 110 so that curled elastic member 110 can be locally bent at an edge of the part where grip portion 144 is attached to curled elastic member 110. In any constitution, it is necessary for bending portion 113 to have a restoring force for restituting the bending portion from the bending state to the original annular state.

According to the first to fifth embodiments, the sticker, finger-hitching member, grip portion and the like were described as the examples of the indicator, however, the present invention is not limited to the mentioned examples. For example, the indicator can be arranged in such manner that the surface of the cover is colored differently to the other parts, or an additional cloth material is stitched to constitute the indicator. Any indicator merely capable of informing the user of the spot to be held by hand in the fitting operation can be adopted.

Further, the first to fourth embodiments were described referring to the examples in which, as shown in Figs. 2, 10, 12 and 14, sticker 141, finger-hitching members 142 and 143, and grip portion 144 serving as the indicator for showing the spot to be held by hand in the fitting operation are respectively provided on the surface of the cuff at the position corresponding to region D opposing to overlapping region C of curled elastic member 110 and the region closer to one end 111 of curled elastic member 110 than opposing region D. However, sticker 141, finger-hitching members 142 and 143 and grip portion 144 as the indicator may be respectively provided on the surface of outer cloth 131 of cover 130 only at the position corresponding to region D opposing to overlapping region C of curled elastic member 110. Alternatively, sticker 141, finger-hitching members 142 and 143 and grip portion 144 as the indicator may be respectively provided on the surface of outer cloth 131 of cover 130 only at the position corresponding to the region closer to one end 111 of curled elastic member 110 than opposing region D. Further, sticker 141, finger-hitching members 142 and 143 and grip portion 144 as the indicator are preferably provided on the surface of outer cloth 131 of cover 130 at the position corresponding to the region closer to one end 111 than central position E (see Figs. 2, 10, 12 and 14) in the circumferential direction of curled elastic member 110 extending from one end 111 to the other end 112 thereof. When sticker 141, finger-hitching members 142 and 143 and grip portion 144 are provided in the foregoing position, the large bending moment can be reliably obtained in the fitting operation, and curled elastic member 110 can be thereby more easily changed in size allowing the winding operation to be more facilitated.

Further, the second to fifth embodiments were described referring to the curled elastic member having the overlapping region in which the circumferential one end and the other end are overlapped with each other in the non-fitting state. However, the one end and the other end are not necessarily overlapped with each other, and the present invention can be applied to the constitution in which the clearance for inserting the arm is provided between the one end and the other end in the circumferential direction in the non-fitting state as recited in the description of cuff 100A' for blood pressure monitor (see Fig. 7), which was introduced as a modification example in the first embodiment.

Further, the first to fifth embodiments were described based on the cuff for blood pressure monitor designed in such manner that the cuff is wound around the upper arm of the left hand. However, the cuff may be designed in such manner as to be wound around the upper arm of the right hand. The cuff is not necessarily wound around the upper arm, and can be naturally designed for application to a forearm, wrist, lower extremity or the like.

Although the present invention has been described and illustrated in detail, it is clearly understood that the same is by way of illustration and example only and is not to be taken by way of limitation, the scope of the present invention being limited only by the terms of the appended claims.

## Claims

1. A cuff for blood pressure monitor wound around a living body to be fitted thereon, comprising:
a fluid bag (120) for pressing the living body;
an elastic member (110) wound annularly on the outside of said fluid bag (120) and changeable in size in a radial direction; and
a cover (130) having a bag shape for including said fluid bag (120) and said elastic member (110) and having a velcro fastener (133) for fastening and securing said cuff, wherein
said elastic member (110) has an overlapping region (C) in which one end (111) and the other end (112) thereof in a circumferential direction are overlapped with each other so that said one end (111) is located on an inner side of said other end (112) in a non-fitting state, **characterized in that**
an indicator for showing a spot to be held by hand in the fitting operation is provided at a position corresponding to a region (D) opposing to said overlapping region (C) of said elastic member (110) and/or a region closer to said one end (111) of said elastic member (110) than the opposing region (D) in the non-fitting state, and
said indicator is implemented by a finger-hitching member (142) attached to a surface of said cuff, said finger-hitching member (142) having a recessed portion (142a) for receiving a finger in a surface thereof.

2. The cuff for blood pressure monitor according to claim 1, wherein
said recessed portion (142a) extends in a direction intersecting the circumferential direction of said elastic member (110) extending from said one end (111) to said other end (112) thereof.

3. The cuff for blood pressure monitor according to claim 1, wherein
said finger-hitching member (142) is formed from elastomer.

4. The cuff for blood pressure monitor according to claim 1, wherein
said finger-hitching member (142) is provided at a position closer to one end in an axial direction of the surface of said cuff.

5. A cuff for blood pressure monitor wound around a living body to be fitted thereon, comprising:
a fluid bag (120) for pressing the living body;
an elastic member (110) wound annularly on the outside of said fluid bag (120) and having one end (111) and the other end (112) thereof in a circumferential direction, said elastic member being changeable in size in a radial direction; and
a cover (130) having a bag shape for including said fluid bag (120) and said elastic member (110) and having a velcro fastener (133) for fastening and securing said cuff, **characterized in that**
an indicator for showing a spot to be held by hand in the fitting operation is provided at a position corresponding to a region closer to said one end (111) of said elastic member (110) than a central position (E) thereof in the circumferential direction extending from said one end (111) to said other end (112), and
said indicator is implemented by a finger-hitching member (142) attached to a surface of said cuff, said finger-hitching member (142) having a recessed portion (142a) for receiving a finger in a surface thereof.

6. A cuff for blood pressure monitor wound around a living body to be fitted thereon, comprising:
a fluid bag (120) for pressing the living body;
an elastic member (110) wound annularly on the outside of said fluid bag (120) and having one end (111) and the other end (112) thereof in a circumferential direction, said elastic member being changeable in size in a radial direction; and
a cover (130) having a bag shape for including said fluid bag (120) and said elastic member (110) and having a velcro fastener (133) for fastening and securing said cuff, wherein
said elastic member (110) has a bendable bending portion (113), **characterized in that**
an indicator for showing a spot to be held by hand in the fitting operation is provided at a position corresponding to a region closer to said one end (111) of said elastic member (110) than said bending portion (113) of said elastic member (110), and
said indicator is implemented by a finger-hitching member (142) attached to a surface of said cuff, said finger-hitching member (142) having a recessed portion (142a) for receiving a finger in a surface thereof.

## Patentansprüche

1. Manschette für einen Blutdruckmesser, die um einen lebenden Körper zur Anlage daran gewickelt wird, aufweisend:
einen Fluidbeutel (120) zur Druckbeaufschlagung des lebenden Körpers;
ein elastisches Element (110), das ringförmig um die Außenseite des Fluidbeutels (120) gewickelt und das in radialer Richtung in dessen Größe veränderbar ist; und
eine Abdeckung (130), die eine Beutelform zum Aufnehmen des Fluidbeutels (120) und des elastischen Elements (110) und einen Klettverschluss (133) zum Fixieren und Sichern der Manschette aufweist, wobei
das elastische Element (110) einen Überlappungsbereich (C) aufweist, in dem dessen eines Ende (111) und dessen anderes Ende (112) in einer Umfangsrichtung derart überlappen, dass das eine Ende (111) an einer Innenseite des anderen Endes (112) in einem Nicht-Anlage-Zustand angeordnet ist, **dadurch gekennzeichnet, dass**
ein Indikator zum Anzeigen einer bei dem Anlegevorgang mit der Hand zu haltenden Stelle an einer Position vorgesehen ist, die in dem Nicht-Anlage-Zustand einem Bereich (D), der dem Überlappungsbereich (C) des elastischen Elements (110) gegenüberliegt, und/oder einem Bereich, der näher an dem einen Ende (111) des elastischen Elements (110) als der gegenüberliegende Bereich (D) ist, entspricht, und
wobei der Indikator als ein Fingereinhakelement (142), das an einer Fläche der Manschette angebracht ist, ausgeführt ist, wobei das Fingereinhakelement (142) einen Aussparungsabschnitt (142a) zur Aufnahme eines Fingers in dessen Oberfläche aufweist.

2. Manschette für einen Blutdruckmesser gemäß Anspruch 1, wobei
sich der Aussparungsabschnitt (142a) in einer Richtung erstreckt, die die Umfangsrichtung des elastischen Elements (110), das sich von dem einen Ende (111) zu dem anderen Ende (112) erstreckt, schneidet.

3. Manschette für einen Blutdruckmesser gemäß Anspruch 1, wobei das Fingereinhakelement (142) aus einem Elastomer ausgebildet ist.

4. Manschette für einen Blutdruckmesser gemäß Anspruch 1, wobei das Fingereinhakelement (142) an einer Position vorgesehen ist, die in axialer Richtung näher an einem Ende der Fläche der Manschette ist.

5. Manschette für einen Blutdruckmesser, die um einen lebenden Körper zur Anlage daran gewickelt wird, aufweisend:
einen Fluidbeutel (120) zur Druckbeaufschlagung des lebenden Körpers;
ein elastisches Element (110), das ringförmig um die Außenseite des Fluidbeutels (120) gewickelt ist und ein Ende (111) und ein anderes Ende (112) in einer Umfangsrichtung aufweist, wobei das elastische Element in radialer Richtung in dessen Größe veränderbar ist; und
eine Abdeckung (130), die eine Beutelform zum Aufnehmen des Fluidbeutels (120) und des elastischen Elements (110) und einen Klettverschluss (133) zum Fixieren und Sichern der Manschette aufweist, **dadurch gekennzeichnet, dass**
ein Indikator zum Anzeigen einer bei dem Anlegevorgang mit der Hand zu haltenden Stelle an einer Position vorgesehen ist, die einem Bereich entspricht, der näher an dem einen Ende (111) des elastischen Elements (110) als dessen Mittelposition (E) in der Umfangsrichtung, die sich von dem einen Ende (111) zu dem anderen Ende (112) erstreckt, ist, und
wobei der Indikator als ein Fingereinhakelement (142), das an einer Fläche der Manschette angebracht ist, ausgeführt ist, wobei das Fingereinhakelement (142) einen Aussparungsabschnitt (142a) zur Aufnahme eines Fingers in dessen Oberfläche aufweist.

6. Manschette für einen Blutdruckmesser, die um einen lebenden Körper zur Anlage daran gewickelt wird, aufweisend:
einen Fluidbeutel (120) zur Druckbeaufschlagung des lebenden Körpers;
ein elastisches Element (110), das ringförmig um die Außenseite des Fluidbeutels (120) gewickelt wird und ein Ende (111) und ein anderes Ende (112) in einer Umfangsrichtung aufweist, wobei das elastische Element in einer radialen Richtung in dessen Größe veränderbar ist; und
eine Abdeckung (130), die eine Beutelform zur Aufnahme des Fluidbeutels (120) und des elastischen Elements (110) und einen Klettverschluss (133) zum Fixieren und Sichern der Manschette aufweist, wobei
das elastische Element (110) einen biegbaren Biegeabschnitt (113) aufweist, **dadurch gekennzeichnet, dass**
ein Indikator zum Anzeigen einer bei dem Anlegevorgang mit der Hand zu haltenden Stelle an einer Position vorgesehen ist, die einem Bereich entspricht, der näher an dem einen Ende (111) des elastischen Elements (110) als der Biegeabschnitt (113) des elastischen Elements (110) ist, und
wobei der Indikator als ein Fingereinhakelement (142), das an einer Fläche der Manschette angebracht ist, ausgeführt ist, wobei das Fingereinhakelement (142) einen Aussparungsabschnitt (142a) zur Aufnahme eines Fingers in dessen Fläche aufweist.

## Revendications

1. Brassard occlusif destiné à surveiller la pression sanguine, enroulé autour d'un corps vivant et adapté à celui-ci, qui comprend :
une poche de fluide (120) destinée à presser le corps vivant ;
un élément élastique (110) enroulé de manière annulaire sur l'extérieur de ladite poche de fluide (120) et dont la taille peut être modifiée dans une direction radiale ; et
une couverture (130) ayant une forme de poche pour inclure ladite poche de fluide (120) et ledit élément élastique (110) et ayant une fixation Velcro (133) pour attacher et fixer ledit brassard occlusif, dans lequel
ledit élément élastique (110) a une région de chevauchement (C) dont une extrémité (111) et l'autre extrémité (112) se chevauchent l'une l'autre dans une direction circonférentielle de manière à ce que l'une desdites extrémités (111) se situe sur un côté intérieur de l'autre desdites extrémités (112) dans un état non ajusté, **caractérisé en ce que**
un indicateur destiné à indiquer un point à tenir à la main durant l'opération d'ajustement est fourni à une position correspondant à une région (D) opposée à ladite région de chevauchement (C) dudit élément élastique (110) et/ou une région plus proche de l'une desdites extrémités (111) dudit élément élastique (110) que la région opposée (D) dans l'état non ajusté, et
ledit indicateur est mis en application par un élément de fixation du doigt (142) attaché à une surface dudit brassard occlusif, ledit élément de fixation du doigt (142) ayant une partie encastrée (142a) destinée à recevoir un doigt dans une de ses surfaces.

2. Brassard occlusif destiné à surveiller la pression sanguine selon la revendication 1, dans lequel
ladite partie encastrée (142a) s'étend dans une direction coupant la direction circonférentielle dudit élément élastique (110) s'étendant de l'une desdites extrémités (111) à l'autre desdites extrémités (112) de celui-ci.

3. Brassard occlusif destiné à surveiller la pression sanguine selon la revendication 1, dans lequel
ledit élément de fixation du doigt (142) est formé par un élastomère.

4. Brassard occlusif destiné à surveiller la pression sanguine selon la revendication 1, dans lequel
ledit élément de fixation du doigt (142) est fourni à une position plus proche d'une extrémité dans une direction axiale de la surface dudit brassard occlusif.

5. Brassard occlusif destiné à surveiller la pression sanguine, enroulé autour d'un corps vivant et adapté à celui-ci, qui comprend :
une poche de fluide (120) destinée à presser le corps vivant ;
un élément élastique (110) enroulé de manière annulaire sur l'extérieur de ladite poche de fluide (120) et ayant l'une de ses extrémités (111) et l'autre de ses extrémités (112) dans une direction circonférentielle, la taille dudit élément élastique pouvant être modifiée dans une direction radiale ; et
une couverture (130) ayant une forme de poche pour inclure ladite poche de fluide (120) et ledit élément élastique (110) et ayant une fixation Velcro (133) pour attacher et fixer ledit brassard occlusif, **caractérisé en ce que**
un indicateur destiné à indiquer un point à tenir à la main durant l'opération d'ajustement est fourni à une position correspondant à une région plus proche de l'une desdites extrémités (111) dudit élément élastique (110) que d'une position centrale (E) de celui-ci dans la direction circonférentielle s'étendant de l'une desdites extrémités (111) à l'autre desdites extrémités (112), et
ledit indicateur est mis en application par un élément de fixation du doigt (142) attaché à une surface dudit brassard occlusif, ledit élément de fixation du doigt (142) ayant une partie encastrée (142a) destinée à recevoir un doigt dans une de ses surfaces.

6. Brassard occlusif destiné à surveiller la pression sanguine, enroulé autour d'un corps vivant et adapté à celui-ci, qui comprend :
une poche de fluide (120) destinée à presser le corps vivant ;
un élément élastique (110) enroulé de manière annulaire sur l'extérieur de ladite poche de fluide (120) et ayant l'une de ses extrémités (111) et l'autre de ses extrémités (112) dans une direction circonférentielle, la taille dudit élément élastique pouvant changer dans une direction radiale ; et
une couverture (130) ayant une forme de poche pour inclure ladite poche de fluide (120) et ledit élément élastique (110) et ayant une fixation Velcro (133) pour attacher et fixer ledit brassard occlusif, dans lequel
ledit élément élastique (110) possède une partie flexible pliante (113), **caractérisé en ce que**
un indicateur destiné à indiquer un point à tenir à la main durant l'opération d'ajustement est fourni à une position correspondant à une région plus proche de l'une desdites extrémités (111) dudit élément élastique (110) que ladite partie flexible (113) dudit élément élastique (110), et
ledit indicateur est mis en application par un élément de fixation du doigt (142) attaché à une surface dudit brassard occlusif, ledit élément de fixation du doigt (142) ayant une partie encastrée (142a) destinée à recevoir un doigt dans une de ses surfaces.
